# EUROPEAN PATENT APPLICATION

(11) **EP 2 388 616 A2**
(43) Date of publication of application: **23.11.2011**
(21) Application number: 11150633.3
(22) Date of filing: 11.01.2011
(51) Int. Cl.: G01S 7/52

(54) **Display system and method of ultrasound apparatus**

(30) Priority: 17.05.2010 KR 20100046041
(71) Applicant: Samsung Medison Co., Ltd., Nam-myun Hongchun-gun Kangwon do (KR)
(72) Inventor: Kim, Sung Yoon, Gyeonggi-do 472-735 (KR); Lee, Kwang Hee, Daejeion 302-743 (KR)
(74) Representative: Schmid, Wolfgang

(57) **Abstract**

A display system and method of a 3-dimensional (3D) ultrasound apparatus is provided. A display system of a 3D ultrasound apparatus includes a first display unit and a second display unit. The first display unit scans a side image of an object in a human body and displays the side image in a first area on a screen. The second display unit scans a top image of the object and displays the top image in a second area different from the first area on the screen.

## Description

### CROSS-REFERENCE TO RELATED APPLICATION

This application claims the benefit of Korean Patent Application No. 2010-0046041, filed on May 17, 2010 in the Korean Intellectual Property Office, the disclosure of which is incorporated herein by reference.

### BACKGROUND

### 1. Field of the Invention

The present invention relates to a display system and method of a 3-dimensional (3D) ultrasound apparatus for scanning and displaying side and top images of an object in a human body.

### 2. Description of the Related Art

An ultrasound system is an apparatus that irradiates an ultrasound signal from a surface of a human body towards a target part, for example, an object such as a fetus, an internal organ, and the like, within the body surface and obtains an image of a monolayer or blood flow in soft tissue from information in the reflected ultrasound signal. The ultrasound system has been widely used together with other image diagnostic systems such as X-ray diagnostic systems, computerized tomography (CT) scanners, magnetic resonance image (MRI) systems and nuclear medicine diagnostic systems because of various merits such as a small size, a low price, real-time image display, and high stability through elimination of any radiation exposure.

Also, a general method for diagnosing Down's syndrome in a fetus is to measure the thickness of the fetus' nuchal translucency (NT) through the ultrasound system. The method was developed by Nicolaides in 1992, and it has been known that where body fluid is accumulated in subcutaneous tissues at the nape of a fetus' neck, the fetus' NT of the fetus becomes thick. Particularly, in the case of a fetus having hetero chromosomes causing Down's syndrome or a heart abnormality, the fetus' NT becomes thick in most cases. Therefore, a doctor measures the thickness of a translucent part at the back of a fetus' neck. In a case where the thickness of the part exceeds 2.5 mm, the doctor inspects an abnormality of the fetus using a more precise method such as a chorionic villus sampling or amniocentesis.

Another method for diagnosing Down's syndrome in a fetus is to measure the angle between the fetus' maxilla and the ridge of the fetus' nose, that is, a frontomaxillary facial (FMF) angle. For example, in a case where the FMF angle of a fetus is more than 88.7 degrees as compared with 78.1 that is the FMF angle of a normal fetus, it is highly likely that the fetus has Down's syndrome.

Therefore, an apparatus for more easily and precisely recognizing and displaying the thickness of fetus NT or the angle between the fetus' maxilla and the ridge of the fetus' nose is desired to easily diagnose a fetus afflicted with Down's syndrome.

### SUMMARY

An aspect of the present invention provides a display system and method of a 3-dimensional (3D) ultrasound apparatus, in which the display system of the 3D ultrasound apparatus scans and displays a side image of an object in a human body, and in a case where the object is a fetus and the side image is a sagittal view, the display system of the 3D ultrasound apparatus displays either the frontomaxillary facial (FMF) angle between the fetus' maxilla and the ridge of the fetus' nose, or the thickness of fetus' nuchal translucency (NT), so that the FMF angle and the thickness of the NT may be more easily and precisely recognized.

An aspect of the present invention also provides a display system and method of an ultrasound apparatus, in which the display system of the 3D ultrasound apparatus scans and displays a top image of an object in a human body, and in a case where the object is a fetus and the side image is a sagittal view, the display system of the 3D ultrasound apparatus overlaps characteristics, for example, the biparietal diameter (BPD), occipitofrontal diameter (OFD), head circumference (HC), a ratio between the BPD and OFD, and the like, of a figure matched to the circumference of the fetus' head with the top image and displays the characteristics, so that the characteristics of the fetus' head may be precisely recognized.

According to an aspect of the present invention, there is provided a display system of a 3D ultrasound apparatus, the system including a first display unit to scan a side image of an object in a human body and to display the side image in a first area on a screen, and a second display to scan a top image of the object and to display the top image in a second area different from the first area on the screen.

According to an aspect of the present invention, there is provided a display method of a 3D ultrasound apparatus, the method including scanning a side image of an object in a human body and displaying the side image in a first area on a screen, and scanning a top image of the object and displaying the top image in a second area different from the first area on the screen.

According to embodiments of the present invention, a display system of a 3D ultrasound apparatus scans and displays a side image of an object in a human body, and in a case where the object is a fetus and the side image is a sagittal view, the display system of the 3D ultrasound apparatus displays either the FMF angle between the fetus' maxilla and the ridge of the fetus' nose, or the thickness of the fetus' NT, so that the FMF angle and the thickness of the NT may be more easily and precisely recognized.

Also, according to embodiments of the present invention, a display system of a 3D ultrasound apparatus scans and displays a top image of an object in a human body, and in a case where the object is a fetus and the side image is a sagittal view, the display system of the 3D ultrasound apparatus overlaps characteristics, for example, the BPD, OFD, HD, a ratio between the BPD and OFD, and the like, of a figure matched to the circumference of the fetus' head with the top image and displays the same, so that the characteristics of the fetus' head may be precisely recognized.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
- FIG. 1: is a block diagram illustrating a configuration of a display system of a 3-dimensional (3D) ultrasound apparatus according to an embodiment of the present invention;
- FIG. 2: is a diagram illustrating an example of an image of an object displayed in the display system of the 3D ultrasound apparatus according to the embodiment of the present invention; and
- FIG. 3: is a flowchart illustrating a display method of the 3D ultrasound apparatus according to an embodiment of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

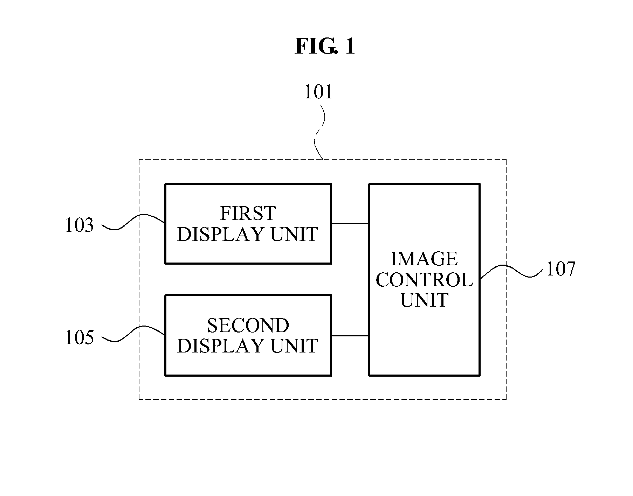

FIG. 1 is a block diagram illustrating a configuration of a display system 101 of a 3-dimensional (3D) ultrasound apparatus according to an embodiment of the present invention.

Referring to FIG. 1, the display system 101 of the 3D ultrasound apparatus according to the present embodiment includes a first display unit 103, a second display unit 105 and an image control unit 107.

The first display unit 103 scans a side image of an object in a human body and displays the side image in a first area on a screen. Here, the object in the human body may be a fetus or an internal organ.

Also, the first display unit 103 may extract any one of side planes, that is, A-planes of the object, scanned in a side direction, as the side image. Here, the A-planes of the object are a plurality of side images periodically scanned in the side direction according to a predetermined time interval. The A-planes of the object may be different images from one another according to the movement of the object.

The first display unit 103 may scan a side image of the object rotated by the image control unit 107 at a selected interval. As the scanned side image is a sagittal view, the first display unit 103 may display information for informing that the scanned side image is the sagittal view in the first area when the rotation of the object is stopped by the image control unit 107.

In a case where the object is a fetus and the side image is a sagittal view, the first display unit 103 may display, in the first area, either the frontomaxillary facial (FMF) angle between the fetus' maxilla and the ridge of the fetus' nose, measured by an FMF measuring unit of the 3D ultrasound apparatus, or the thickness of fetus' nuchal translucency (NT), measured by an NT measuring unit of the 3D ultrasound apparatus. Accordingly, an operator (or doctor) can more precisely diagnose where the fetus has an abnormal symptom based on the measured FMF angle or thickness of the fetus' NT.

Here, the FMF measuring unit specifies a first significant point related to the ridge of the fetus' nose and a second significant point related to the fetus' maxilla, and measures the angle between lines that pass through the first and second significant points, respectively, thereby obtaining the FMF angle. Also, the FMF measuring unit may find the first and second significant points by using a selected algorithm and image processing for an ultrasound data or by using experience over time and accumulated data according to the experience points.

Accordingly, the first display unit 103 may overlap portions and the FMF angle of the lines that pass through the respective first and second significant points specified by the FMF measuring unit, and the outline and thickness of the fetus' NT with the side image and then display the same in the first area.

The second display unit 105 scans a top image of the object and displays the top image in a second area different from the first area on the screen. Here, the second display unit 105 may extract any one of top planes, for example, in a case where the object is a fetus, planes of a fetus' head, viewed from the top. Here, the top planes may be B-planes of the object, scanned in a top direction perpendicular to the side direction, as the top image. Here, the B-planes of the object are a plurality of side images periodically scanned in the top direction according to a predetermined time interval. The B-planes of the object may be different images from each other depending on the movement of the object.

In a case where the object is a fetus and the side image is a sagittal view, the second display unit 105 may overlap a figure, for example, an ellipse, and the like, matched to the circumference of the top image with the top image and then display the same in the second area. The second display unit 105 may display a center point of the figure together with the figure. Here, the center point of the figure is a center point of the object, and may be extracted by the FMF measuring unit based on the first significant point related to the ridge of the fetus' nose and the second significant point related to the fetus' maxilla.

Here, the second display unit 105 may display, in the second area, at least one of the biparietal diameter (BPD), occipitofrontal diameter (OFD) and head circumference (HD) of the figure and the ratio of the BPD and OFD, measured by a head measuring unit of the 3D ultrasound apparatus.

The second display unit 105 displays a reference figure related to the circumference together with the figure matched to the circumference, so that a difference between the figure matched to the circumference and the reference figure may be easily recognized. For example, in a case where the object is a fetus, the second display unit 105 displays a figure matched to the circumference of the normal fetus' head together with a figure matched to the circumference of the scanned fetus' head, so that a difference between the circumference of the fetus' head to be inspected and the circumference of the normal fetus' head may be distinctly represented.

Also, in a case where the object is a fetus and the side image is a sagittal view, the second display unit 105 may overlap, for example, the circumference of the fetus' head and the figure matched to the face circumference of the fetus as the circumference of the top image with the top image and display the same in the second area.

The image control unit 107 may set a virtual axis that passes through an arbitrary point, for example, a center point, and the like, of the B-plane of the object, related to the top image, and an inside, for example, a middle, and the like, of the A-plane of the object, related to the side image, and rotate the object in a selected direction with respect to the virtual axis based on a control command. Here, the image control unit 107 may rotate the object with the minimum rotation in the direction in which the side image of the object is a sagittal view.

For example, in a case where the side image of the object is a sagittal view, the image control unit 107 may control the object to be rotated by 20 degrees in a first direction. Since the image control unit 107 controls the object to be rotated by 340 degrees in a second direction, the first direction with the minimum rotation may be more effective than the second direction.

The image control unit 107 controls the first display unit 103 to scan a side image of the rotated object at a selected interval. In a case where the scanned side image is a sagittal view, the image control unit 107 may stop the rotation of the object.

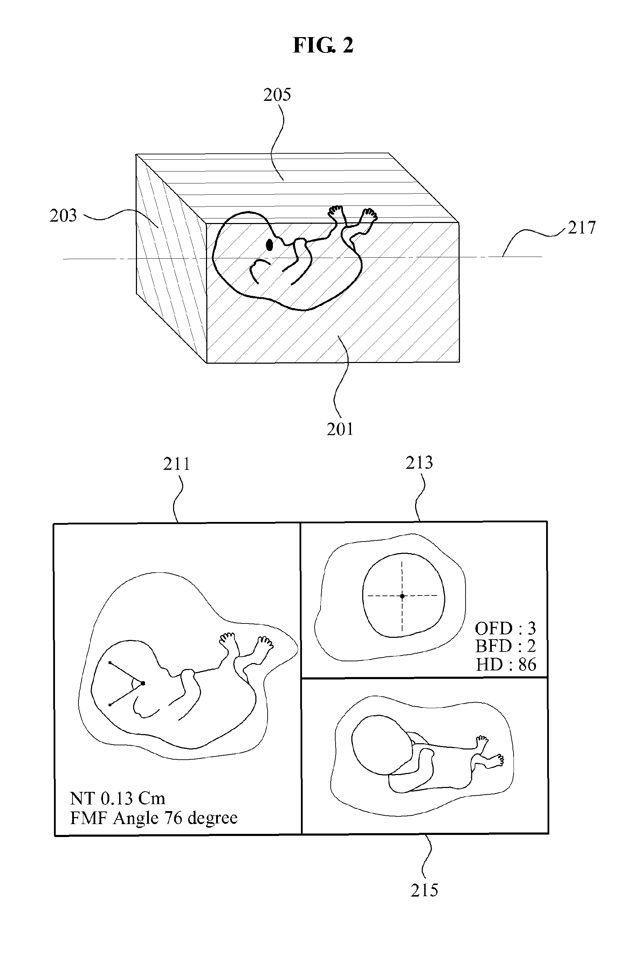

FIG. 2 is a diagram illustrating an example of an image of an object displayed in the display system of the 3D ultrasound apparatus according to the embodiment of the present invention.

Referring to FIG. 2, the display system of the 3D ultrasound apparatus may scan side and top images of an object in a human body and display the scanned side and top images in first and second areas on a screen, respectively.

For example, the display system of the 3D ultrasound apparatus may scan a side image of an object in a side direction that views a 'first plane' 201 and display the scanned image in a first area 211 on a screen. The display system of the 3D ultrasound apparatus may scan a top image of the object in a top direction that views a 'second plane' 203 and display the scanned image in a second area 213 on the screen.

The display system of the 3D ultrasound apparatus may scan a top image of the object in a top direction that views a 'third plane' 205 and display the scanned image in a third area 215 on the screen.

Also, in a case where the object is a fetus and the side image is a sagittal view, the display system of the 3D ultrasound apparatus may display, in the first area 211, at least one of a first significant point related to the ridge of the fetus' nose and a second significant point related to the fetus' maxilla, the FMF angle between the fetus' maxilla and the ridge of the fetus' nose, and the outline and thickness of the fetus' NT.

In a case where the object is a fetus and the side image is a sagittal view, the display system of the 3D ultrasound apparatus may overlap a figure matched to the circumference of the top image scanned in the top direction that views the 'second plane' 203 with the top image and display the same in the second area 213. Here, the display system of the 3D ultrasound apparatus may display, in the second area 213, at least one of the BPD, OFD, HD and the ratio of the BPD and OFD of the circumference of the top image, that is, at least one of the BPD, OFD, HD and the ratio of the BPD and OFD of the figure matched with the circumference of the fetus' head.

In a case where the object is a fetus and the side image is a sagittal view, the display system of the 3D ultrasound apparatus may overlap a figure matched to the circumference of the top image scanned in the top direction that views the 'third plane' 205 with the top image and display the same in the third area 215. Here, the display system of the 3D ultrasound apparatus may display, in the third area 215, at least one of the BPD, OFD, HD and the ratio of the BPD and OFD of the circumference of the top image, that is, at least one of the BPD, OFD, HD and the ratio of the BPD and OFD of the figure matched with the face circumference of the fetus.

The center point of the figure displayed in the second and third areas 213 and 215 is a center point of the object, and may be extracted based on the first significant point related to the ridge of the fetus' nose and the second significant point related to the fetus' maxilla.

In a case where the side image does not correspond to a sagittal view, the display system of the 3D ultrasound apparatus may control the side image to be the sagittal view by setting a virtual axis 217 that passes through the center point of the B-plane of the object, related to the top image, and the middle of the A-plane of the object, related to the side image, and rotating the object in a selected direction with respect to the virtual axis 217 based on a control command.

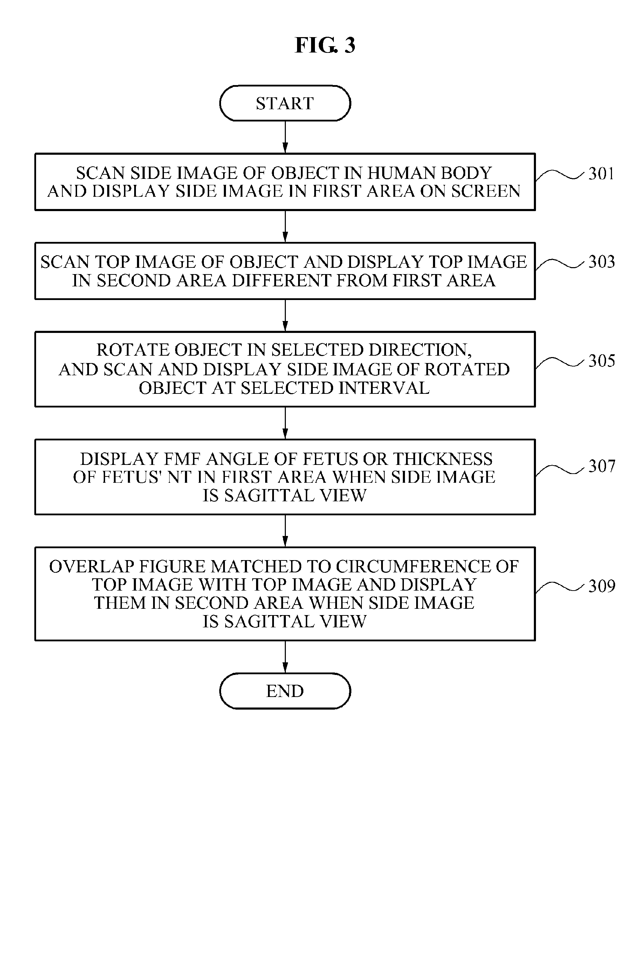

FIG. 3 is a flowchart illustrating a display method of the 3D ultrasound apparatus according to an embodiment of the present invention.

Referring to FIG. 3, the display system of the 3D ultrasound apparatus scans a side image of an object in a human body and displays the side image in a first area on a screen in operation 301.

The display system of the 3D ultrasound apparatus may extract and display any one of A-planes of the object scanned in a side direction as the side image.

The display system of the 3D ultrasound apparatus scans a top image of the object and displays the top image in a second area different from the first area on the screen in operation 303.

The display system of the 3D ultrasound apparatus may extract and display any one of B-planes of the object scanned in a top direction perpendicular to the side direction as the top image.

The display system of the 3D ultrasound apparatus rotates the object in a selected direction, and scans and displays a side image of the rotated object at a selected interval in operation 305.

Here, the display system of the 3D ultrasound apparatus may set a virtual axis that passes through the center point of the B-plane of the object, related to the top image, and the middle of the A-plane of the object, related to the side image, and rotate the object in the selected direction with respect to the virtual axis based on a control command. Here, the display system of the 3D ultrasound apparatus scans the side image of the rotated object at the selected interval. In a case where the scanned side image is a sagittal view, the display system of the 3D ultrasound apparatus may stop the rotation of the object.

In a case where the scanned side image is a sagittal view, the display system of the 3D ultrasound apparatus displays information for informing that the scanned side image is the sagittal view in the first area when the rotation of the object is stopped.

In a case where the object is a fetus and the side image is a sagittal view, the display system of the 3D ultrasound apparatus may display, in the first area, either the FMF angle between the fetus' maxilla and the ridge of the fetus' nose, measured by the FMF measuring unit of the 3D ultrasound apparatus, or the thickness of fetus' NT, measured by the NT measuring unit of the 3D ultrasound apparatus in operation 307.

In a case where the object is a fetus and the side image is a sagittal view, the display system of the 3D ultrasound apparatus may overlap a figure matched to the circumference of the top image with the top image and display them in the second area in operation 309.

Here, the display system of the 3D ultrasound apparatus may display, in the second area, at least one of the BPD, OFD, and HD of the figure and the ratio of the BPD and OFD, measured by the head measuring unit of the 3D ultrasound apparatus.

According to embodiments of the present invention, a display system of a 3D ultrasound apparatus scans and displays a side image of an object in a human body, and in a case where the object is a fetus and the side image is a sagittal view, the display system of the 3D ultrasound apparatus displays either the FMF angle between the fetus' maxilla and the ridge of the fetus' nose, or the thickness of fetus' NT, so that the FMF angle and the thickness of the NT may be more easily and precisely recognized.

Also, according to embodiments of the present invention, a display system of a 3D ultrasound apparatus scans and displays a top image of an object in a human body, and in a case where the object is a fetus and the side image is a sagittal view, the display system of the 3D ultrasound apparatus overlaps characteristics, for example, the BPD, OFD, HD, a ratio between the BPD and OFD, and the like, of a figure matched with the circumference of the fetus' head with the top image and displays the same, so that the characteristics of the fetus' head may be precisely recognized.

The above-described exemplary embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. A display system of a 3-dimensional (3D) ultrasound apparatus, the system comprising:
a first display unit to scan a side image of an object in a human body and to display the side image in a first area on a screen; and
a second display to scan a top image of the object and to display the top image in a second area different from the first area on the screen.

2. The system of claim 1, wherein the first display unit extracts any one of A-planes of the object scanned in a side direction as the side image, and the second display unit extracts any one of B-planes of the object scanned in a top direction perpendicular to the side direction as the top image.

3. The system of claim 1, further comprising an image control unit to set a virtual axis that passes through an arbitrary point in the B-plane of the object, related to the top image, and an inside of the A-plane of the object, related to the side image, and to rotate the object in a selected direction with respect to the virtual axis based on a control command.

4. The system of claim 3, wherein the image control unit controls the first display unit to scan a side image of the rotated object in a selected interval, and stops the rotation of the object when the scanned side image is a sagittal view.

5. The system of claim 4, wherein the first display unit displays information for informing that the scanned side image is the sagittal view in the first area when the rotation of the object is stopped.

6. The system of claim 1, wherein, when the object is a fetus and the side image is a sagittal view, the first display unit displays, in the first area, at least one of the frontomaxillary facial (FMF) angle between the fetus' maxilla and the ridge of the fetus' nose, measured by an FMF measuring unit of the 3D ultrasound apparatus, and the thickness of fetus' nuchal translucency (NT), measured by an NT measuring unit of the 3D ultrasound apparatus.

7. The system of claim 1, wherein, when the object is a fetus and the side image is a sagittal view, the second display unit overlaps a figure matched to the circumference of the top image with the top image and displays the figure and the top image in the second area.

8. The system of claim 7, wherein the second display unit displays, in the second area, at least one of the biparietal diameter (BPD), occipitofrontal diameter (OFD) and head circumference (HD) of the figure and the ratio between the BPD and OFD, measured by a head measuring unit of the 3D ultrasound apparatus.

9. A display method of a 3D ultrasound apparatus, the method comprising:
scanning a side image of an object in a human body and displaying the side image in a first area on a screen; and
scanning a top image of the object and displaying the top image in a second area different from the first area on the screen.

10. The method of claim 9, wherein the displaying of the side image in the first area on the screen comprises extracting and displaying any one of A-planes of the object scanned in a side direction as the side image, and the displaying of the top image in the second area different from the first area on the screen comprises extracting and displaying any one of B-planes of the object scanned in a top direction perpendicular to the side direction as the top image.

11. The method of claim 9, further comprising setting a virtual axis that passes through an arbitrary point in the B-plane of the object, related to the top image, and an inside of the A-plane of the object, related to the side image, and rotating the object in a selected direction with respect to the virtual axis based on a control command.

12. The method of claim 11, further comprising scanning a side image of the rotated object in a selected interval, and stopping the rotation of the object when the scanned side image is a sagittal view.

13. The method of claim 12, further comprising displaying information for informing that the scanned side image is the sagittal view in the first area when the rotation of the object is stopped.

14. The method of claim 9, further comprising, when the object is a fetus and the side image is a sagittal view, displaying, in the first area, at least one of the frontomaxillary facial (FMF) angle between the fetus' maxilla and the ridge of the fetus' nose, measured by an FMF measuring unit of the 3D ultrasound apparatus, and the thickness of fetus' nuchal translucency (NT), measured by an NT measuring unit of the 3D ultrasound apparatus.

15. The method of claim 9, further comprising, when the object is a fetus and the side image is a sagittal view, overlapping a figure matched to the circumference of the top image with the top image and displaying the figure and the top image in the second area.
